# EUROPÄISCHE PATENTSCHRIFT

(11) **EP 0 563 585 B1**
(45) Veröffentlichungstag und Bekanntmachung des Hinweises auf die Patenterteilung: **09.08.1995**
(21) Anmeldenummer: 93103204.9
(22) Anmeldetag: 01.03.1993
(51) Int. Cl.: A61B 17/16

(54) **Knochenraspel aus Kunststoff**
Plastic bone rasp
Râpe à os en plastique

(30) Priorität: 01.04.1992 CH 1043/92
(43) Veröffentlichungstag der Anmeldung: 06.10.1993
(73) Patentinhaber: IMT INTEGRAL MEDIZINTECHNIK AG, CH-6373 Ennetbürgen (CH)
(72) Erfinder: Geisser, Albert, CH-6373 Ennetbürgen (CH); Becker, Hubertus, W-8921 Apfeldorf (DE); Elmiger, Heinz, CH-6102 Malters (CH)
(74) Vertreter: Blum, Rudolf Emil Ernst

(56) Entgegenhaltungen:
- EP-A- 0 136 079
- EP-A- 0 331 626
- EP-A- 0 405 132
- CH-A- 611 509
- DE-A- 3 907 256
- DE-U- 8 601 685
- FR-A- 2 274 267
- FR-A- 2 503 988
- US-A- 4 751 922

## Beschreibung

Die Erfindung betrifft eine chirurgische Raspel zur Bearbeitung von Knochen beim operativen Einsatz künstlicher Gelenkteile, mit einem gezahnten Bearbeitungsteil und einem Adapterteil, welches zur Befestigung der Raspel an einem Schlagwerkzeug bestimmt ist gemäß dem Oberbegriff des Anspruchs 1 wie es beispielsweise aus der EP-A-331626 bekannt ist.

Zur Bearbeitung der Lagerflächen von Knochen zur Aufnahme eines künstlichen Gelenkteiles werden bekannterweise Raspeln aus Metall oder Metalllegierungen verwendet, z.B. Raspeln aus Stahl, rostfreiem Stahl, oberflächenbehandeltem Stahl, chemisch vernickeltem, oder hartverchromtem Stahl. Solche Raspeln sind in verschiedenen Grössen und Formen bekannt, z.B. gemäss der EP-A-331 626 und der EP-A-136 079, welche zudem auf Raspeln aus weicheren Metallen verweist. Diese Raspeln werden nach jeder Operation gereinigt und mittels Sterilisationsgeräten wiederum steril aufbereitet. Das Wiederaufbereiten ist in bezug auf das Kontaminationsrisiko problematisch. So sind die Raspeln nach der Operation mit Blut und Knochen behaftet und müssen mit Bürsten von Hand gereinigt werden. Diese Arbeit ist unangenehm und durch die geschliffenen oder gehauenen scharfen Kanten der Raspeln besteht Verletzungsgefahr. Bei der Handhabung der Raspeln bei der Sterilisation und der Reinigung besteht ferner die Gefahr, dass die Raspeln mit anderen Metallteilen in Berührung kommen und dadurch stumpf werden. Eine Abstumpfung ergibt sich natürlich auch durch die mehrfache Verwendung dieser Raspeln. Auch eine geringfügige Stumpfheit der Raspelzähne wirkt sich indes beim Gebrauch nachteilig aus. Ferner sind die bekannten Raspeln aus Metall sehr schwer, was deren Handhabung bei der Operation erschwert.

Aus DE-U-8 601 685.7 ist ein zahnärztliches Werkzeug zur Wurzelbehandlung bekannt, bei dem eine Förderspirale aus Kunststoff gebildet ist.

Der Erfindung liegt die Aufgabe zugrunde, eine Raspel zur Bearbeitung von Knochen zu schaffen, welche diese Nachteile nicht aufweist. Dies wird bei einer Raspel der eingangs genannten Art dadurch erreicht, dass der Bearbeitungsteil mit den Zähnen aus Kunststoff besteht.

Bevorzugterweise besteht auch der Adapterteil der Raspel aus Kunststoff.

Durch eine solche Raspel, welche zumindest teilweise, vorzugsweise aber vollständig aus Kunststoff besteht, werden die genannten Probleme gelöst. Bei einer solchen Raspel, welche kostengünstig herstellbar ist, kann nämlich auf die mehrmalige Verwendung verzichtet werden. Die Raspel wird also nach einmaligem Gebrauch weggeworfen und muss daher nicht gereinigt und nicht sterilisiert werden. Die Raspel wird vorzugsweise bereits steril verpackt geliefert und erst kurz vor ihrer Verwendung aus ihrer Verpackung genommen. Als weiterer Vorteil ergibt sich, dass die Raspel aus Kunststoff sehr leicht ist, was deren Handhabung bei der Operation erleichtert.

Im folgenden werden Ausführungsbeispiele der Erfindung anhand der Zeichnungen näher erläutert. Dabei zeigt
Figur 1 eine teilweise geschnittene Ansicht der Breitseite einer Raspel;
Figur 2 eine teilweise geschnittene Ansicht der Schmalseite der Raspel von Figur 1;
Figur 3 eine Ansicht der Breitseite der mittleren Bearbeitungszone der Raspel von Figur 1;
Figur 4 eine Ansicht der Schmalseite der mittleren Bearbeitungszone der Raspel;
Figur 5 eine Ansicht der mittleren Berarbeitungszone einer Raspel mit Doppelverzahnung;
Figur 6 eine Ansicht der Schmalseite der mittleren Bearbeitungszone der Raspel mit Doppelverzahnung.

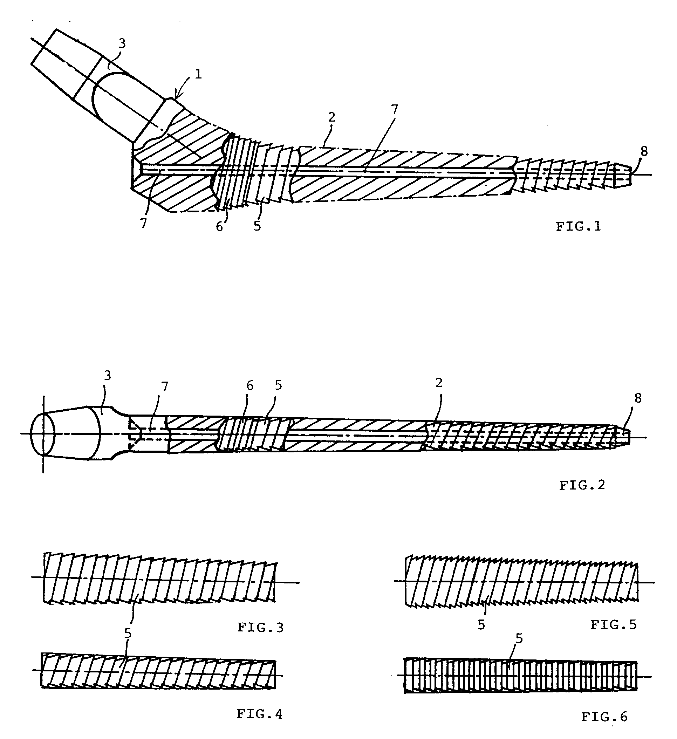

Figur 1 zeigt die Breitseite einer Knochenraspel 1 aus Kunststoff. Dabei besteht mindestens der gezahnte Bearbeitungsteil 2 der Raspel aus Kunststoff. Vorzugsweise ist aber auch der Adapterteil 3 aus Kunststoff gefertigt, so dass die ganze Raspel aus Kunststoff besteht. Der Adapterteil 3 ist dazu bestimmt, die Raspel an ein Schlagwerkzeug zu koppeln, welches die Bearbeitungsschläge zur Bearbeitung des Knochens erzeugt. Ein solches Bearbeitungswerkzeug, welches pneumatisch angetrieben wird, ist z.B. aus der europäischen Patentanmeldung 452 543 bekannt. Ein derartiges Bearbeitungswerkzeug ist für die Verwendung mit Kunststoffraspeln besonders geeignet, da die hochfrequenten, maschinellen Impulse dieses Bearbeitungswerkzeugs im Vergleich zu herkömmlichen Hammer- oder Gleithammerschlägen klein sind, so dass eine Ueberbeanspruchung der Kunststoffraspel sicher ausgeschlossen wird.

Beim erstmaligen Einsetzen einer Prothese, vor allem aber auch bei einem Prothesenwechsel, wenn das Implantat zementiert war, ist das Säubern des Knochens durch Spülen von zentraler Bedeutung für den Erfolg der Operation. Bei der Bearbeitung mit der Raspel werden alte Zementrückstände, Bindegewebe und Knochenlamellen freigeschabt und ausgekratzt und mittels Spülung und Absaugung wird das Knochenbett zur Aufnahme des neuen Kunstgelenkes präpariert. Bei den bekannten Raspeln wird dieser Spül- und Absaugvorgang erst im Nachhinein, nach dem Entfernen der Raspeln mittels Küretten oder anderen Instrumenten vorgenommen. Bevorzugterweise wird nun bei der Kunststoffraspel eine Bohrung 7 vorgesehen, welche am proximalen Ende der Raspel an eine Spül- und Absaugleitung angeschlossen werden kann und welche am distalen Ende der Raspel 8 offen ist. So kann der Raspelvorgang, das Spülen und das Absaugen der zu entfernenden Zement-, Knochen- und Spongiosaresten in einem Arbeitsgang durchgeführt werden. Gleichzeitig wird durch diese Spülung während des Raspelns eine Kühlung im Knochen erreicht. Diese Kühlung ist sinnvoll, weil durch das Eintreiben der Raspel eine Wärmeentwicklung entstehen kann, die eine Hitzenekrose von aktivem Knochen hervorrufen kann.

Vorzugsweise weist die Raspel einen Bearbeitungsteil 2 auf, bei welchem Verzahnungen mit verschiedenen Steigungen vorgesehen sind. Bei der in Figur 1 gezeigten Raspel ist der vordere und mittlere Teil der Bearbeitungszone mit einer ersten Verzahnung 5 versehen. Der hintere Teil der Bearbeitungszone, in welchen sich die Raspel in der Breite stark aufweitet, ist mit einer feineren Verzahnung 6 versehen. Dadurch wird sowohl ein rasches als auch ein schonendes Bearbeiten des Knochens möglich.

Figur 2 zeigt die Ansicht der Schmalseite der Raspel von Figur 1, wobei gleiche Bezugszeichen gleiche Elemente wie in Figur 1 bezeichnen. In Figur 3 und Figur 4 ist die Verzahnung 5 des mittleren Bearbeitungsteils dargestellt. Figur 5 und Figur 6 zeigen eine weitere Ausführungsform der Verzahnung. Dabei ist die Schmalseite der Raspel mit einer Doppelverzahnung versehen.

Als Kunststoffmaterial für die Raspel kommen verschiedene Kuntstoffe in Frage, zum Beispiel Polyoximethylen (POM), Polyamid (PA) und Polyethylen (PE), ferner LCP (Liquid cristalline polymers; Flüssigkristallpolymere) oder PVDF (Polyvinylidenfluoride).

Die genannten Kunststoffe können ferner durch die Zugabe von Kohlenstoff-Fasern verstärkt werden. Besonders bevorzugt von den genannten Kunststoffen sind Polyoximethylen oder Polycarbonat, insbesondere der Kunststoff vom Typ Macrolon 2805 der Firma Bayer. Die Herstellung der Raspel kann durch Spritzgiessen, durch Warmumformung oder durch mechanische Bearbeitung eines Rohlings erfolgen.

## Patentansprüche

1. Chirurgische Raspel zur Bearbeitung von Knochen beim operativen Einsatz künstlicher Gelenkteile mit einem gezahnten Bearbeitungsteil und mit einem Adapterteil, welches zur Befestigung der Raspel an einem Schlagwerkzeug bestimmt ist, dadurch gekennzeichnet, dass der Bearbeitungsteil (2) mit den Zähnen aus Kunststoff besteht.

2. Raspel nach Anspruch 1, dadurch gekennzeichnet, dass der Adapterteil (3) aus Kunststoff besteht.

3. Raspel nach Anspruch 1 oder 2, dadurch gekennzeichnet, dass die Raspel (1) mit einer sich in Längsrichtung erstreckenden Bohrung (7) versehen ist.

4. Raspel nach einem der vorangehenden Ansprüche, dadurch gekennzeichnet, dass der Bearbeitungsteil (2) mit Zonen (5,6) unterschiedlicher Steigung der Verzahnung versehen ist.

5. Raspel nach einem der Ansprüche 1 bis 4, dadurch gekennzeichnet, dass der eine Breitseite und eine Schmalseite aufweisende Bearbeitungsteil an der Schmalseite eine Doppelzahnung aufweist.

6. Raspel nach einem der Ansprüche 1 bis 5, dadurch gekennzeichnet, dass als Kunststoff ein Polycarbonat-Kunststoff vorgesehen ist.

7. Raspel nach einem der Ansprüche 1 bis 5, dadurch gekennzeichnet, dass als Kunststoff Polyoximethylen (POM) vorgesehen ist.

8. Raspel nach einem der Ansprüche 1 bis 7, dadurch gekennzeichnet, dass der Kunststoff mit Kohlenstoff-Fasern verstärkt ist.

## Claims

1. Surgical rasp for working bone during operative implantation of artificial joint parts, with a toothed working part and with an adapter part destined for fixing the rasp to an impact tool characterised in that the working part (2) with the teeth consists of plastic.

2. Rasp of claim 1 characterised in that the adapter part (3) consists of plastic.

3. Rasp of claim 1 or 2 characterised in that the rasp (1) is provided with a bore (7) extending in longitudinal direction.

4. Rasp of one of the preceding claims characterised in that the working part (2) is provided with zones (5, 6) of different slope of the toothing.

5. Rasp of one of the claims 1 to 4 characterised in that the working part having a broad side and a narrow side comprises a double toothing at the narrow side.

6. Rasp of one of the claims 1 to 5 characterised in that a polycarbonate plastic is provided as the plastic.

7. Rasp of one of the claims 1 to 5 characterised in that the plastic is a polyoximethylene (POM).

8. Rasp of one of the claims 1 to 7 characterised in that the plastic is reinforced with carbon fibres.

## Revendications

1. Râpe chirurgicale pour le façonnage des os lors de l'insertion d'éléments d'articulation artificiels, avec une partie dentée servant au façonnage et une partie d'accouplement servant à fixer la râpe à un instrument de percussion, caractérisée en ce que la partie dentée (2) servant au façonnage est réalisée en plastique.

2. Râpe selon la revendication 1, caractérisée en ce que la partie d'accouplement (3) est réalisée en plastique.

3. Râpe selon la revendication 1 ou 2, caractérisée en ce que la râpe (1) comporte un passage longitudinal (7).

4. Râpe selon une des revendications précédentes, caractérisée en ce que la partie dentée (2) servant au façonnage comporte des zones (5, 6) dont les dents ont des angles d'attaque différents.

5. Râpe selon une des revendications 1 à 4, caractérisée en ce que la partie dentée présente un côté large et un côté étroit, et que ce dernier possède une denture double.

6. Râpe selon une des revendications 1 à 5, caractérisée en ce que le plastique est un polycarbonate.

7. Râpe selon une des revendications 1 à 5, caractérisée en ce que le plastique est un polyoxyméthylène (POM).

8. Râpe selon une des revendications 1 à 7, caractérisée en ce que le plastique est renforcé par des fibres de carbone.
